# FASCICULE DE BREVET EUROPEEN

(11) **EP 2 689 750 B1**
(45) Date de publication et mention de la délivrance du brevet: **24.08.2016**
(21) Numéro de dépôt: 13177477.0
(22) Date de dépôt: 22.07.2013
(51) Int. Cl.: A61F 2/40, A61F 2/30, A61B 17/86

(54) **Implant glénoidien pour prothèse d'épaule et kit chirurgical**
Glenoidales Implantat für Schulterprothese und chirurgisches Kit
Glenoidal implant for shoulder prosthesis and surgical kit

(30) Priorité: 23.07.2012 US 201261674702 P; 18.09.2012 FR 1258748
(43) Date de publication de la demande: 29.01.2014
(73) Titulaire: Tornier Orthopedics Ireland Ltd., Macroom (IE)
(72) Inventeur: Cardon, Jean-Emmanuel, 38330 Biviers (FR); Fenlin, John M., Bryn Mawr, PA 19010 (US); Nicholson, Grégory P., Western Springs, IL 60558 (US); Burkhead, Wayne, Dallas 75231 (US)
(74) Mandataire: Lavoix

(56) Documents cités:
- EP-A1- 1 336 396
- FR-A1- 2 652 498
- FR-A1- 2 737 107
- FR-A1- 2 871 369
- US-A1- 2009 204 225

## Description

La présente invention concerne un implant glénoïdien pour prothèse d'épaule. L'invention concerne également un kit chirurgical adapté pour la mise en oeuvre d'un implant glénoïdien. Le domaine de l'invention est celui des prothèses d'épaule, comprenant un implant glénoïdien destiné à être implanté dans la glène de l'omoplate d'un patient.

De manière classique, un tel implant glénoïdien comprend un corps articulaire adapté pour l'articulation avec la tête de l'humérus, prothésée ou non. En pratique, l'implant glénoïdien peut être conformé selon une configuration anatomique, dans laquelle le corps articulaire comporte une cavité prévue pour remplacer la cavité glénoïdale, ou bien selon une configuration inversée, dans laquelle le corps articulaire comporte un dôme hémisphérique prévu pour coopérer avec une cavité complémentaire définie par un implant huméral. Le corps articulaire peut être métallique, par exemple en alliage de titane, en céramique, ou synthétique, par exemple en polyéthylène.

EP-A-2 481 376 décrit différents modes de réalisation d'un tel implant glénoïdien, présentant une grande polyvalence. L'implant comprend un insert de fixation du corps articulaire à la glène. L'insert est ancré à demeure dans la glène par le chirurgien, avant le positionnement du corps articulaire sur cet insert. L'implant comprend également des moyens de blocage de la rotation de l'insert et du corps articulaire par rapport à la glène.

FR-A-2 737 107 décrit différents modes de réalisation d'un autre implant glénoïdien, comprenant un corps articulaire; une platine de fixation à la glène, une rondelle et une vis. Lorsque l'implant est assemblé, ces différents éléments sont centrés par rapport à un axe d'ancrage. Le corps articulaire est centré sur une surface externe de la platine de fixation, la rondelle est centrée contre une surface interne du corps articulaire et la vis traversant la rondelle est centrée dans la platine de fixation. La vis forme des moyens de blocage de la rotation de la rondelle par rapport à la platine. La position de la rondelle peut être réglée en rotation autour de l'axe d'ancrage, mais pas en translation transversalement à l'axe d'ancrage.

Dans certains cas, le chirurgien peut être amené à réviser l'épaule avec une nouvelle prothèse, anatomique ou inversée. L'insert reste à demeure dans la glène, tandis que le corps articulaire est remplacé. Les implants existants permettent difficilement au chirurgien de mettre en oeuvre un nouveau corps articulaire d'un type différent du précédent, par exemple de convertir une configuration anatomique en configuration inversée. En outre, lorsque le chirurgien veut réviser la glène avec un implant inversé, le dôme glénoïdien doit généralement être décalé du côté inférieur de la glène par rapport à la position précédemment définie par l'insert. Autrement dit, le centre articulaire en configuration anatomique peut être différent du centre articulaire en configuration inversée. A cet effet, il existe des gammes de corps articulaires présentant différentes excentrations de la surface articulaire. Cependant, les corps articulaires sont des éléments coûteux, cette solution n'est donc pas entièrement satisfaisante.

Le but de la présente invention est de proposer un implant glénoïdien amélioré, notamment en termes de polyvalence, simplicité et coût de mise en oeuvre.

A cet effet, l'invention a pour objet un implant glénoïdien pour prothèse d'épaule, destiné à être implanté dans la glène d'un patient et comprenant les caractéristiques de la revendication 1.

Ainsi, l'invention permet d'améliorer la polyvalence, la simplicité et le coût de mise en oeuvre de l'implant glénoïdien, qui peut présenter une configuration anatomique ou inversée en fonction des besoins du chirurgien. Les moyens d'ancrage peuvent rester à demeure dans l'implant, de sorte que la révision de la prothèse d'épaule est facilitée. Les moyens d'ajustement permettent un positionnement précis de la platine et donc du corps articulaire par rapport aux moyens d'ancrage et à la glène, quelle que soit la configuration choisie pour l'implant. En particulier, l'excentration du corps articulaire par rapport aux moyens d'ancrage est réglée au niveau de la platine et des moyens d'ajustement, ce qui évite de devoir prévoir une gamme importante de corps articulaires coûteux. La platine peut être positionnée au meilleur emplacement sur la glène, en fonction de la configuration différentes configurations anatomiques et osseuses à la fois grâce à un ajustement transversal, notamment suivant une direction supérieure-inférieure, et un ajustement en rotation, notamment en fonction de la configuration particulière de la glène du patient. En outre, un ajustement de la platine par basculement par rapport à l'axe d'ancrage peut être de préférence effectué dans le cas où la surface de la glène n'est pas perpendiculaire à l'axe d'ancrage. L'implant selon l'invention, avantageusement modulaire et convertible, peut se présenter sous forme d'un kit chirurgical, comme détaillé ci-après.

Selon d'autres caractéristiques avantageuses de l'implant glénoïdien selon l'invention, prises isolément ou en combinaison :
- Les moyens d'ajustement définissent de plus une liaison rotule pour ajuster en position la platine, préalablement à son blocage en position par les moyens de blocage, en basculement par rapport à l'axe d'ancrage.
- Les moyens d'ajustement comprennent une rainure ménagée dans la platine suivant une direction transverse à l'axe d'ancrage et adaptée pour ajuster en position la platine transversalement à l'axe d'ancrage indépendamment d'une rotation de cette platine autour de l'axe d'ancrage.
- La rainure comporte au moins deux empreintes délimitant chacune une position distincte de la platine transversalement à l'axe d'ancrage indépendamment d'une rotation de cette platine autour de l'axe d'ancrage.
- Les moyens d'ajustement comprennent un pion coopérant avec les moyens d'ancrage et la platine pour ajuster en position cette platine par rapport à l'axe d'ancrage.
- Le pion coopère avec une rainure ménagée dans la platine pour ajuster en position cette platine par rapport à l'axe d'ancrage.
- Le pion reçu dans l'une des empreintes formées dans la rainure bloque en position la platine transversalement à l'axe d'ancrage indépendamment d'une rotation de cette platine autour de l'axe d'ancrage.
- Le corps articulaire présente sélectivement une configuration glénoïdienne anatomique ou inversée.

L'invention a également pour objet un kit chirurgical selon les caractéristiques de la revendication 9.

Selon d'autres caractéristiques avantageuses du kit chirurgical selon l'invention, prises isolément ou en combinaison :
- Les moyens d'ajustement comprennent une rainure ménagée dans la ou au moins l'une des platines suivant une direction transverse à l'axe d'ancrage et adaptée pour ajuster en position cette platine transversalement à l'axe d'ancrage indépendamment d'une rotation de cette platine autour de l'axe d'ancrage.
- Le kit comprend au moins deux platines ajustables sélectivement selon un ensemble de configurations en rotation autour de l'axe d'ancrage et transversalement à l'axe d'ancrage différent pour chacune de ces platines.

L'invention sera mieux comprise à la lecture de la description qui va suivre, donnée uniquement à titre d'exemple non limitatif et faite en référence aux dessins annexés sur lesquels :
- la figure 1 est une vue en perspective éclatée d'un implant glénoïdien conforme à l'invention, comprenant une platine également conforme à l'invention ;
- la figure 2 est une autre vue en perspective éclatée, selon un angle différent, de l'implant de la figure 1 ;
- la figure 3 est une section partielle de l'implant des figures 1 et 2, implanté dans la glène d'un patient également montrée en section ;
- la figure 4 est une vue en élévation de la platine des figures 1 à 3 ;
- la figure 5 est une vue de côté d'un pion appartenant à l'implant des figures 1 à 3 et permettant d'ajuster la platine en position ;
- la figure 6 est une vue en perspective partielle de l'implant des figures 1 à 3, dans une configuration assemblée ;
- la figure 7 est une vue selon la flèche VII à la figure 6 ;
- les figures 8, 9 et 10 sont des vues analogues à la figure 4, montrant d'autres variantes de platines conforme à l'invention ;
- les figures 11 et 13 sont des vues analogues aux figures 4 et 8 à 10, montrant d'autres variantes de platines adaptées pour équiper un kit chirurgical, et qui ne représentent pas l'invention ; et
- les figures 12 et 14 sont des vues en perspective des platines, respectivement des figures 11 et 13.

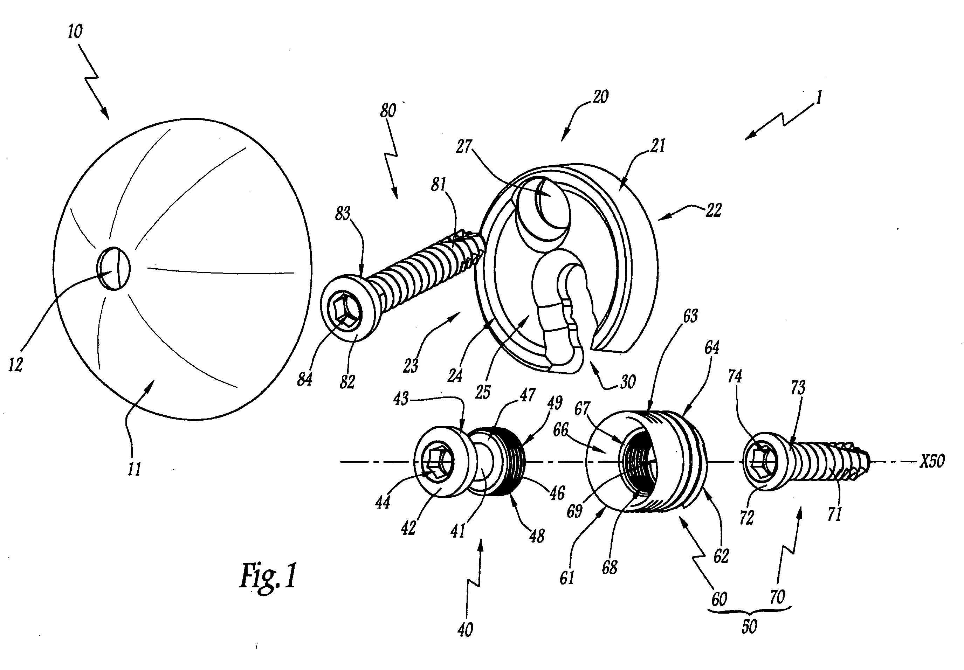

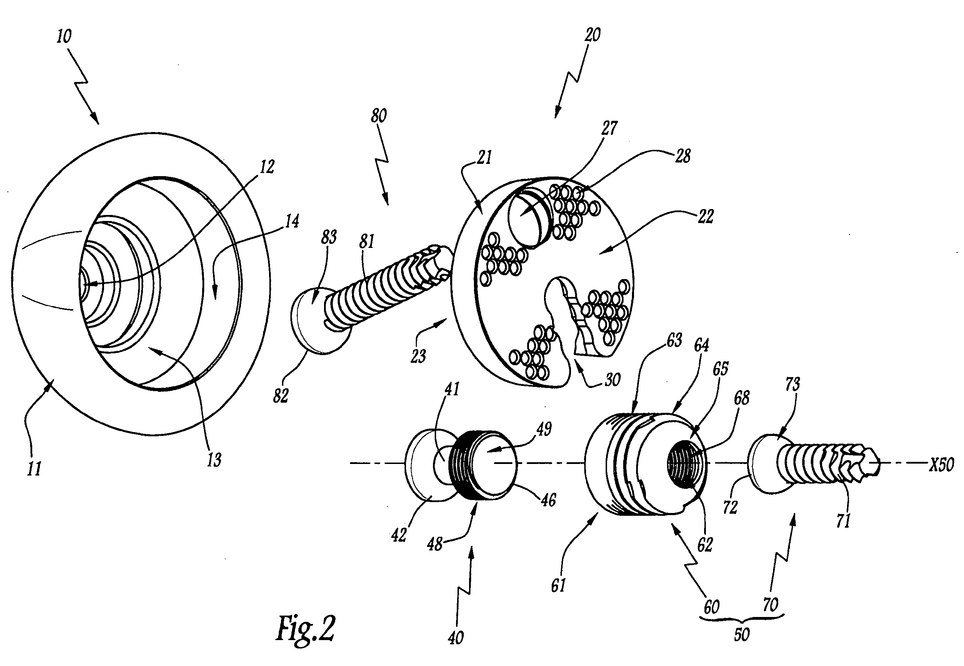

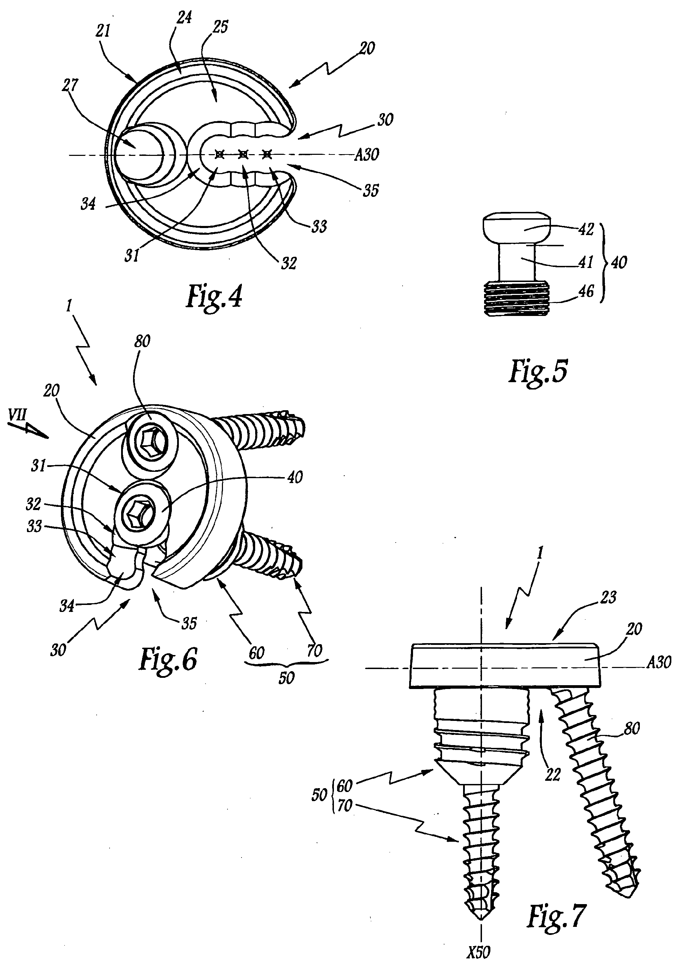

Sur les figures 1 à 7 est représenté un implant glénoïdien 1 conforme à l'invention.

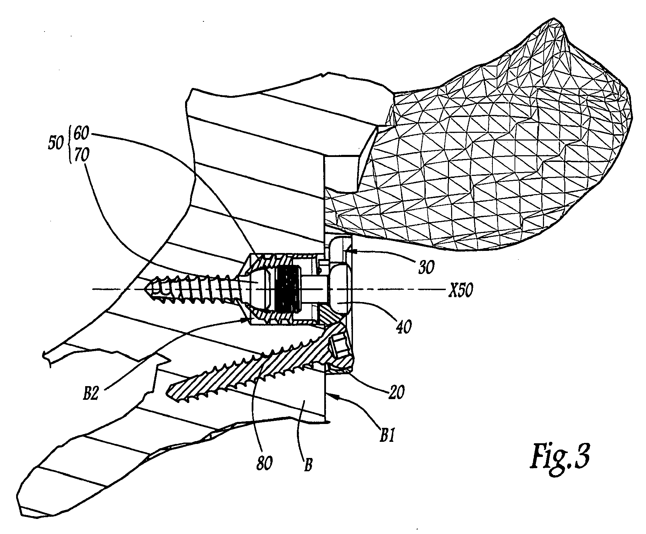

Cet implant 1 est destiné à être implanté dans la glène B de l'omoplate d'un patient. La glène B est montrée uniquement à la figure 3, tandis que l'humérus n'est pas représenté, dans un but de simplification. La glène B comprend une préparation osseuse qui remplace la cavité glénoïdale d'origine, incluant une surface d'appui B1 et une cavité d'ancrage B2. La surface B1 est sensiblement plane, par exemple réalisée par fraisage. La cavité B2 débouche de la glène B au niveau de cette surface B1.

Par la suite, afin de se repérer dans l'espace, on désigne par côté arrière de l'implant 1 les éléments tournés vers la glène B et par côté avant de l'implant 1 les éléments tournés à l'opposé de la glène B, lorsque l'implant 1 est implanté dans la glène B. En termes anatomiques, le côté arrière est donc orienté suivant la direction médiale, tandis que le côté avant est orienté suivant la direction latérale du corps du patient.

L'implant 1 comprend un corps articulaire 10 pour l'articulation de l'implant glénoïdien 1 avec l'humérus prothésé ou non-prothésé. L'implant 1 comprend également une platine 20 de support du corps articulaire 10, facilitant le positionnement précis de ce corps articulaire 10 par rapport à la glène B. L'implant 1 comprend également un pion d'ajustement 40, un système d'ancrage 50 incluant un insert 60 et une vis 70, ainsi qu'une vis de blocage 80. L'insert 60 et la vis 70 sont adaptés pour être fixés à demeure à la glène B, plus précisément dans la cavité d'ancrage B2 suivant un axe d'ancrage X50. Le pion 40 et la vis 80 forment des moyens de blocage en position de la platine 20 par rapport au système d'ancrage 50 et à la glène B. La platine 20 comporte une rainure 30 adaptée pour recevoir le pion 40. La rainure 30 et le pion 40 forment des moyens d'ajustement en position de la platine 20 préalablement à son blocage en position par les moyens de blocage 40 et 80, sélectivement en rotation autour de l'axe d'ancrage X50 et en translation transversalement à l'axe d'ancrage X50.

Les éléments 10, 20, 40, 60, 70 et 80 constitutifs de l'implant 1 peuvent être réalisés en métal, alliage métallique, céramique ou polymère biocompatible, par exemple en acier inoxydable, en titane ou alliage de titane, en alliage cobalt-chrome, en pyrocarbone, en PEEK ou tout autre matériau adapté à la présente application. En outre, chacune de ces éléments peut recevoir tout traitement ou revêtement adapté à la présente application, de manière localisée ou non.

Sur l'exemple des figures, l'implant 1 présente une configuration inversée. Ainsi, le corps articulaire 10 présente une forme de dôme hémisphérique. Le corps 10 comprend une surface articulaire externe 11 de forme sphérique convexe, munie d'un orifice au sommet 12. Le corps 10 comprend également une cavité interne 13, incluant en particulier une surface tronconique 14 de fixation à la platine 20. En alternative, la surface 14 peut avoir un profil cylindrique. L'orifice 12 relie la surface externe 11 et la cavité interne 13. Cet orifice 12 permet au chirurgien d'accéder au pion 40 et à la vis 80 lorsque le corps 10 est fixé sur la platine 20.

La platine de support 20 est distincte du corps articulaire 10, du pion 40 et de l'insert 50. La platine 20 comprend une surface externe tronconique 21 reliant un côté arrière 22 et un côté avant 23 de la platine 20. La surface 21 présente un diamètre plus important du côté arrière 22 que du côté avant 23. La surface 21 est prévue pour coopérer avec la surface 14 du corps articulaire 10, en vue de sa fixation selon un positionnement précis par rapport à la glène B. Du côté avant 23, la platine 20 comporte une bordure avant 24 délimitant un évidement avant 25. La platine 20 comporte un orifice 27 de réception de la vis 80, traversant la platine 20 entre l'évidement 25 du côté avant 23 et le côté arrière 22. La platine 20 comporte des trous 28 répartis du côté arrière 22, pour donner un aspect de surface irrégulier à ce côté arrière 22 et ainsi favoriser la repousse osseuse. Excepté les trous 28, la platine 20 présente une surface sensiblement plane du côté arrière 22, adaptée pour venir en appui contre la surface d'appui B1 de la glène B.

La rainure 30 ménagée dans la platine 20 s'étend suivant un axe de rainure A30, qui est sensiblement perpendiculaire à l'axe X50 lorsque la platine 20 est en position. La rainure 30 débouche à la fois au niveau de la surface 21, du côté arrière 22 et du côté avant 23 de la platine 20. La rainure 30 comporte des empreintes 31, 32 et 33, délimitant des positions distinctes d'ajustement de la platine 20. Une bordure 34 est formée autour des empreintes 31-33 du côté arrière 22 de la platine 20, de sorte que les empreintes 31-33 sont plus étroites du côté arrière 22 et plus larges du côté avant 23 de la rainure 30. La bordure 34 s'étend en arc de cercle autour du centre de chaque empreinte 31-33 et présente une concavité du côté avant 23, adaptée pour recevoir le pion 40. L'empreinte 31 est plus rapprochée du centre de la platine 20 et de l'évidement 25 relativement à la surface 21, l'empreinte 33 est formée au niveau de la surface 21 et de la bordure 24, tandis que l'empreinte 32 est formée entre les empreintes 31 et 33 dans l'évidement 25. Suivant l'axe A30, la rainure 30 est fermée au niveau de l'empreinte 31 et munie d'une ouverture transverse 35 délimitée au niveau de la surface 21 et de l'empreinte 33.

Le pion 40 permet d'ajuster la position de la platine 20 par rapport au système d'ancrage 50. Le pion 40 comprend une tige cylindrique 41, une tête 42 comportant une surface arrière bombée 43 et un orifice hexagonal 44, ainsi qu'un pied fileté 46 comportant une surface plane annulaire avant 47, un filetage 48 et une surface plane arrière 49. La tige 41 s'étend entre la surface 43 de la tête 42 et la surface 47 du pied 46. L'orifice 44 est prévu pour recevoir un outil, tel qu'un tournevis chirurgical. Le pied 46 peut ainsi être vissé dans le système d'ancrage 50 suivant l'axe d'ancrage X50. Pour sa part, la tête 41 peut venir se loger dans les empreintes 31-33 et bloquer la position de la platine 20. Ainsi, comme détaillé ci-après, le pion 40 appartient à la fois aux moyens d'ajustement et aux moyens de blocage de la platine 20.

Le système 50 d'ancrage de l'implant 1 dans la glène B est formé par l'insert 60 et la vis 70 et s'étend suivant l'axe d'ancrage X50. Plus précisément, l'insert 60 et la vis 70 sont positionnés à demeure dans la cavité d'ancrage B2 prévue à cet effet. Selon une variante non représentée, l'insert 60 et la vis 70 peuvent constituer une pièce unique. De préférence, dans un cas idéal, l'axe X50 est sensiblement perpendiculaire à la surface d'appui B1. L'insert 60 est globalement en forme de cylindre creux à section circulaire, comportant une ouverture avant 61 et une ouverture arrière 62. L'insert 60 est conformé comme une cheville, pour réaliser une fonction de centrage de l'implant 1 dans la cavité B2 de la glène B. L'insert 60 comporte une surface externe bosselée 63, un filetage externe 64, une surface tronconique arrière 65, une surface interne 66, un épaulement interne 67, un filetage interne 68 et une surface interne 69. L'axe d'ancrage est défini principalement par l'insert 60 plutôt que par la vis 70, en particulier défini par le filetage 68 prévu pour recevoir le pion 40.

A l'extérieur de l'insert 60, la surface 63 est munie de bosselages lui procurant un aspect de surface rugueux, pour adhérer aux parois internes de la cavité d'ancrage B2. Le filetage 64 permet d'ancrer l'insert 60 à la glène B par vissage dans la cavité B2. La surface 65 peut venir en butée au fond de la cavité B2, en fonction de l'enfoncement de l'insert 60, mais ce n'est généralement pas le cas.

A l'intérieur de l'insert 60, la surface 66 est ovale et prévue pour recevoir un outil, qui peut venir en appui contre l'épaulement 67, afin de visser l'insert 60 dans la cavité B2. En alternative, la surface 66 peut être polygonale, par exemple hexagonale, ou cylindrique, et dans ce cas l'outil coopère directement avec le filetage 68. Une fois l'insert 60 en place dans la glène B, le filetage 68 est prévu pour recevoir le pied fileté 46 du pion 40, plus précisément le filetage 48. La surface 69 est tournée vers l'ouverture avant 61 et est prévue pour recevoir la vis 70 en appui.

La vis d'ancrage 70 comporte un corps fileté 71 et une tête de vis 72, munie d'une surface arrière bombée 73 et d'un orifice avant hexagonal 74. La surface 73 est prévue pour venir en appui contre la surface 69 de l'insert 60 lorsque le corps fileté 71 pénètre dans la masse osseuse de la glène B. L'orifice 74 est prévu pour recevoir un outil, tel qu'un tournevis chirurgical, pour visser la vis 70 dans la glène B. Idéalement, l'axe de la vis 70 peut être aligné avec l'axe d'ancrage X50 défini par l'insert 60. En pratique, lors du vissage de la vis 70 dans la glène B, l'axe de cette vis 70 peut être plus ou moins incliné par rapport à l'axe d'ancrage X50. Un tel cas correspond notamment à une vis 70 du type directionnel.

La vis de blocage 80 comporte un corps fileté 81 et une tête de vis 82, munie d'une surface arrière bombée 83 et d'un orifice avant hexagonal 84. L'orifice 84 est prévu pour recevoir un outil, tel qu'un tournevis chirurgical, pour visser le corps fileté 81 de la vis 80 dans la glène B. La surface 83 est prévue pour venir en appui contre les bords de l'orifice 27 formé dans la platine 20, lorsque le corps fileté 81 pénètre dans la masse osseuse de la glène B. Du fait de sa configuration, l'orifice 27 est adapté pour recevoir une vis 80 dite « multidirectionnelle », c'est à dire pouvant être positionnée par rapport à la platine 20 et à la glène B suivant différentes directions, au choix du chirurgien. Le vissage de la vis 80 dans l'orifice 27 de la platine 20 permet de bloquer la position de la platine 20, notamment en rotation par rapport à l'axe d'ancrage X50 du système 50 implanté dans la glène B.

En variante non représentée, la vis de blocage multidirectionnelle 80 peut être remplacée ou complétée par des moyens de blocage par expansion. Par exemple, de tels moyens de blocage peuvent comprendre une rondelle qui s'écarte radialement dans l'orifice 27 lors du vissage d'une vis d'expansion, similaire ou non à la vis 80, dans cette rondelle.

Le procédé de mise en oeuvre de l'implant 1 est détaillé ci-après.

Lors de la pose d'un premier implant 1 dans la glène B, le chirurgien prépare la surface d'appui B1 et la cavité d'ancrage B2. Dans ce cadre, le chirurgien peut réaliser la surface B1 avant ou après la cavité B2. Selon l'invention, le premier implant 1 peut comprendre soit directement un corps articulaire inversé 10, soit un corps articulaire anatomique auquel succèdera un corps articulaire inversé 10 lors d'une révision.

Lorsque le chirurgien perce la cavité B2, il fore un trou dont le diamètre est légèrement inférieur au diamètre externe de l'insert 60, notamment du filetage 64. Le chirurgien visse ensuite l'insert 60 dans la cavité 64, au moyen d'un outil, non représenté, équipé d'un embout coopérant avec la surface 66. Le filetage 64 est de préférence auto-taraudant, ce qui lui permet de pénétrer dans la cavité B2 en taraudant la masse osseuse de la glène B. Le chirurgien fait alors un nouveau trou dans la glène B, de diamètre légèrement inférieur au diamètre du corps fileté 71 de la vis 70, en passant une mèche, non représentée, à travers les ouvertures 61 et 62 de l'insert 60, perpendiculairement à la surface B1. La mèche peut être guidée par un outil additionnel, non représenté, agencé à l'intérieur de l'insert 60. Le chirurgien visse ensuite la vis d'ancrage 70 dans le trou qu'il vient de réaliser et dans l'insert 60, à l'aide d'un outil, non représenté, qu'il insère dans l'orifice 74 de la vis 70. En fin de course, la surface 73 de la vis 70 vient en appui contre la surface 69 de l'insert 60. Sur l'exemple des figures, le filetage 64 de l'insert 60 est « à droite » et son vissage s'effectue dans le sens antihoraire, tandis que le filetage 71 de la vis 70 est « à gauche » et son vissage s'effectue dans le sens horaire. Ainsi, une fois l'insert 60 et la vis 70 en place dans la cavité B2, le système d'ancrage 50 est bloqué en rotation et son dévissage en service est évité. De préférence, l'insert 60 est positionné avec un jeu entre l'ouverture avant 61 et l'embouchure de la cavité B2, ce qui permet éventuellement le fraisage d'une nouvelle surface B1 sans fraiser l'insert 60.

Lorsque le chirurgien révise la glène B, en particulier en mettant en oeuvre l'implant 1 selon l'invention, la cavité B2 est déjà formée et le système d'ancrage 50 reste avantageusement en place dans cette cavité B2. Pour sa part, la surface B1 est formée en remplacement de l'ancienne surface de la glène B. Dans ce cas, la surface B1 peut être inclinée par rapport à un plan perpendiculaire à l'axe d'ancrage X50 précédemment défini.

Ensuite, que ce soit une première implantation ou une révision, le chirurgien introduit le pion 40 dans l'insert 60 par l'ouverture 61, puis visse le pied fileté 46 dans le filetage 68 à l'aide d'un outil, non représenté, qu'il insère dans l'orifice 44 ménagé dans la tête 42 du pion 40. A ce stade, la tige 41 et la tête 42 du pion 40 dépassent de la cavité d'ancrage B2 au niveau de la surface d'appui B1. La platine 20 peut alors coopérer avec le pion 40, en se translatant contre la surface d'appui B1, de sorte que la rainure 30 reçoit la tige 41. De préférence, la bordure 34 de la rainure 30 est dimensionnée pour glisser de part et d'autre de la tige 41, contre la surface externe cylindrique de la tige 41. Grâce à la rainure 30, la platine 20 peut à la fois pivoter et se translater par rapport au pion 40 et à l'axe d'ancrage X50.

Ainsi, la rainure 30 et le pion 40 constituent des moyens d'ajustement en position de la platine 20, préalablement à son blocage en position par le pion 40 et la vis 80, sélectivement en rotation autour de l'axe d'ancrage X50 et en translation transversalement à l'axe d'ancrage X50. En particulier, la rainure 30 permet d'ajuster en position la platine 20 transversalement à l'axe X50, avec une excentration donnée de la platine 20 par rapport à l'axe X50, indépendamment d'une rotation de cette platine 20 autour de l'axe X50. Pour une orientation angulaire donnée de la platine 20 et de l'axe A30 autour de l'axe X50, une translation de la platine 20 est autorisée et dans ce cas, chaque empreinte 31-33 délimite une position distincte de la platine 20 transversalement à l'axe X50. Autrement dit, chaque empreinte 31-33 définit un excentrique donné de la platine 20.

De plus, la rainure 30 et le pion 40 constituent avantageusement des moyens d'ajustement en position de la platine 20 en basculement par rapport à l'axe d'ancrage X50. Ce basculement est réalisé selon une liaison du type rotule entre la tête 41 du pion 40 et l'empreinte 31, 32 ou 33 de la rainure 30 dans laquelle est logée cette tête 41. Ainsi, si la surface B1 n'est pas exactement perpendiculaire à l'axe X50, la platine 20 peut basculer par rapport à cet axe 50 pour venir en appui contre cette surface B1.

Ensuite, le chirurgien bloque la position de la platine 20 par rapport au système d'ancrage 50, à l'axe d'ancrage X50 et à la glène B, grâce aux moyens de blocage constitués par le pion 40 et la vis 80. En l'espèce, la surface 43 de la tête 42 est complémentaire des empreintes 31-33 formées dans la rainure 30. Lorsque le chirurgien visse encore le pion 40 dans l'insert 60, la surface 43 vient appuyer contre la bordure 34 en se logeant dans l'une des empreintes 31-33, bloquant ainsi la position de la platine 20 par rapport à l'insert 30. Le chirurgien fait alors un nouveau trou dans la glène B, de diamètre légèrement inférieur au diamètre du corps fileté 81 de la vis 80, en passant une mèche, non représentée, à travers l'orifice 27 de la platine 20, selon une direction inclinée par rapport à la surface B1. Comme pour le trou de la vis 70, la mèche peut être guidée par un outil additionnel, non représenté, agencé à l'intérieur de l'orifice 27. Le chirurgien visse ensuite la vis de blocage 80 dans le trou qu'il vient de réaliser et dans l'orifice 27, à l'aide d'un outil, non représenté, qu'il insère dans l'orifice 84 de la vis 84. En pratique, le pion 40 est bien adapté pour bloquer la platine 20 essentiellement en translation par rapport au système 50, à l'axe 50 et à la glène B. Néanmoins, le chirurgien utilise la vis 80 pour bloquer de manière certaine la rotation de la platine 20. Autrement dit, les moyens de blocage de la platine 20 comprennent au moins le pion 40, de préférence le pion 40 et la vis 80.

Enfin, le chirurgien fixe le corps articulaire 10 sur la platine 20 par impact. En positionnant avec précision la platine 20 et notamment sa surface 21 de réception du corps 10, le chirurgien peut donc positionner la surface articulaire 11 du corps 10 avec précision par rapport à la glène B. A ce stade, l'implant 1 complet est implanté dans la glène B. Grâce à l'invention, l'opération chirurgicale est sûre et le risque de désassemblage des différentes parties de l'implant 1 est limité. Une fois l'opération terminée, le corps articulaire 10 s'articule avec l'humérus en vue de reproduire un comportement articulaire de type prothèse d'épaule inversée sur l'exemple de la figure 3, ou bien de type prothèse d'épaule anatomique lorsque le corps articulaire présente une configuration anatomique.

En outre, l'implant 1 peut se présenter sous forme d'un kit chirurgical. Dans ce cas, le kit chirurgical comprend au moins un corps articulaire et au moins une platine, tels que le corps 10 et la platine 20 représentés sur l'exemple des figures 1 à 7. De préférence, le kit chirurgical comprend plusieurs corps articulaires et/ou plusieurs platines, telles que les platines 20, 120, 220, 320, 420 et 520 décrites ci-dessus et ci-après.

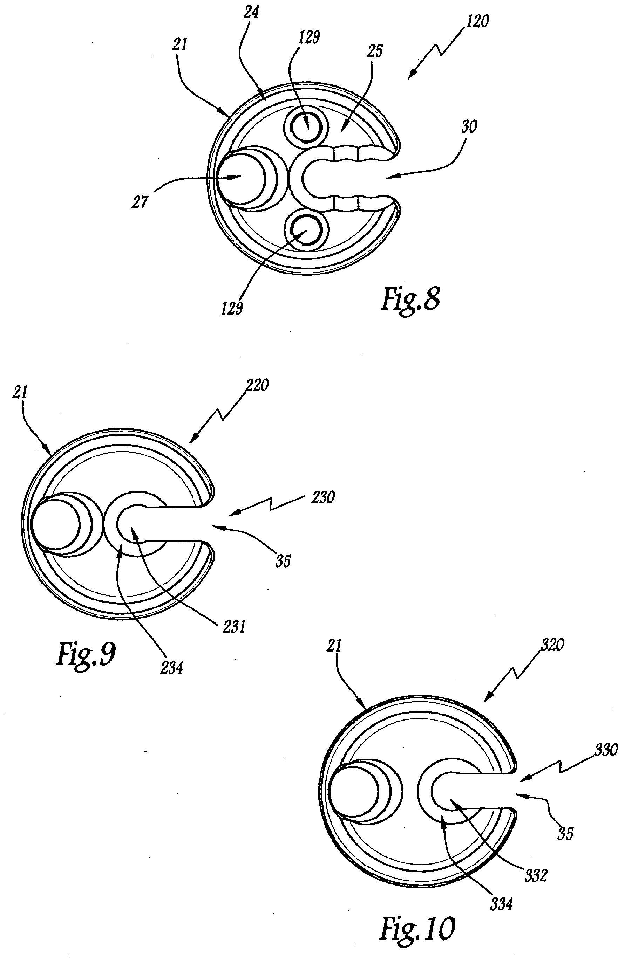

Sur les figures 8, 9 et 10 sont représentées des platines 120, 220 et 320 conformes à d'autres variantes de réalisation de l'invention.

Certaines parties constitutives de ces platines 120, 220 et 320 sont similaires à celles de la platine 20, décrite plus haut, et portent les mêmes références numériques. Seules les différences avec la platine 20 sont détaillées ci-après.

Comme montré à la figure 8, la platine 120 comporte deux orifices traversants additionnels 129, ménagés de part et d'autre de la rainure 30. Ces orifices 129 sont adaptés pour recevoir des moyens de blocage additionnels, tels que des vis similaires à la vis 80 ou tous moyens de blocage adaptés à la présente application.

Comme montré aux figures 9 et 10, les platines 220 et 320 comportent chacune une rainure, respectivement 230 et 330, différente de la rainure 30. En l'espèce, chaque rainure 230 ou 330 comporte une unique empreinte, respectivement 231 ou 332, bordée par une bordure localisée, respectivement 234 ou 334. Les rainures 230 et 330 forment, avec le pion 40 décrit plus haut, des moyens d'ajustement en position de la platine 220 ou 320, préalablement à son blocage en position par les moyens de blocage, sélectivement en rotation autour de l'axe X50, en translation transversalement à l'axe X50 et en basculement par rapport à l'axe X50. Les empreintes 231 et 332 définissent des excentrations distinctes par rapport à leur surface 21 de réception du corps articulaire 10.

Lorsque les platines 220 et 320 équipent le kit chirurgical selon l'invention, le chirurgien peut choisir entre l'une ou l'autre en fonction de l'ajustement en translation désiré par rapport à l'axe d'ancrage X50. Les deux platines 220 et 320 sont ajustables sélectivement selon un ensemble de configurations en rotation autour de l'axe X50 et transversalement à l'axe X50 différent pour chacune de ces platines 220 et 320. Par conséquent, les platines 220 et 320 sont également ajustables sélectivement selon un ensemble de configurations en basculement différent pour chacune d'elle.

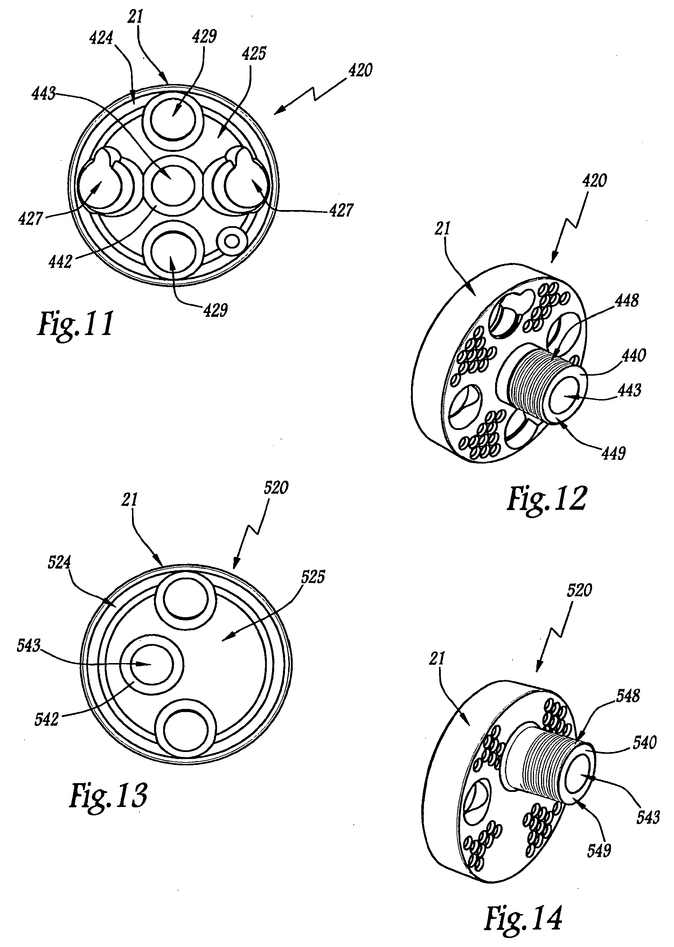

Sur les figures 11 à 14 sont représentées d'autres variantes de platines 420 et 520, adaptées pour équiper un kit chirurgical. Ces variantes ne sont pas conformes à l'invention.

Certaines parties constitutives de ces platines 420 et 520 sont similaires à celles de la platine 20, décrite plus haut, notamment la surface 21 de réception du corps articulaire 10. Seules les différences avec la platine 20 sont détaillées ci-après. En particulier, les platines 420 et 520 ne comportent pas de rainure d'ajustement en position. En revanche, les platines 420 et 520 comportent chacune un pion, respectivement 440 et 540, d'ajustement en position, directement intégré à la platine 420 ou 520 et adapté pour coopérer avec le système d'insert 50 de l'implant 1.

Comme montré aux figures 11 et 12, la platine 420 comprend une bordure 424 et une cavité 425 situées du côté avant, ainsi que deux orifices traversants 427 et deux orifices traversants 429. Les orifices 427 sont globalement similaires à l'orifice 27, tandis que les orifices 429 sont globalement similaires aux orifices 129. Sur l'exemple représenté, les orifices 427 et 429 chevauchent à la fois la bordure 424 et la cavité 425, et sont répartis en alternance à 90° autour du pion 440.

En l'espèce, le pion 440 présente un profil tubulaire cylindrique, centré sur la platine 420 par rapport à la surface 21. Le pion 440 comporte une bordure annulaire avant 442 située dans l'évidement 425, ainsi qu'une bordure annulaire arrière 449. Le pion 440 comporte un alésage cylindrique 443 traversant la platine 420 entre les bordures 442 et 449. Du côté arrière de la platine 420, le pion 440 est plus allongé et comporte un filetage 448 adapté pour coopérer avec le système d'ancrage 50 de l'implant 1. Le pion 440 constitue des moyens d'ajustement en rotation de la platine 420 par rapport à l'axe d'ancrage X50, mais pas en translation. Lorsque l'orientation de la platine 420 par rapport à l'axe X50 varie, la position des orifices 427 et 429 varie, contrairement à la position de la surface 21 de cette platine 420.

Comme montré aux figures 13 et 14, le pion 540 est similaire au pion 440 et comporte les mêmes parties constitutives, portant des références numériques augmentées de 100. Il s'agit des bordures 542 et 549, de l'alésage 543 et du filetage 548, lequel est adapté pour coopérer avec le système d'ancrage de l'implant 1. La platine 520 comprend en outre une bordure 524 et une cavité 525 situées du côté avant, ainsi que deux orifices traversants identiques aux orifices 429.

En l'espèce, la principale différence entre le pion 440 et le pion 540 est que ce pion 540 n'est pas centré par rapport à la surface 21 de la platine 520. Le pion 540 constitue des moyens d'ajustement en rotation de la platine 520 par rapport à l'axe d'ancrage X50, mais pas en translation. Lorsque l'orientation de la platine 520 par rapport à l'axe X50 varie, la position des orifices de passage de vis de blocage varie, mais la position de la surface 21 de cette platine 520 varie également, contrairement à la platine 420. Les configurations possibles de la surface 21 par rapport à la glène B sont donc différentes pour la platine 420 et la platine 520.

En variante non représentée, les pions 420 ou 520 peuvent être configurés différemment en vue de coopérer avec le système d'ancrage 50, par exemple avec un dispositif de crémaillère, un élément expansif ou un circlips.

Indépendamment l'une de l'autre, chacune des platines 420 ou 520 peut être ajustée en position, préalablement à son blocage en position par les moyens de blocage, en rotation autour de l'axe d'ancrage X50, mais pas en translation transversalement à l'axe d'ancrage X50, ni en basculement par rapport à l'axe d'ancrage X50. Lorsque les platines 420 et 520 équipent le kit chirurgical selon l'invention, le chirurgien peut choisir entre l'une ou l'autre en fonction de l'ajustement en translation désiré par rapport à l'axe X50, autrement dit l'excentration de la surface 21 par rapport à l'axe X50. Les deux platines 420 et 520 sont donc ajustables sélectivement selon un ensemble de configurations en rotation autour de l'axe X50 et transversalement à l'axe X50. Cet ensemble de configurations est différent pour chacune de ces platines 420 et 520.

Par ailleurs, l'implant 1 et/ou la platine équipant cet implant 1 peuvent être conformés différemment des figures sans sortir du cadre de l'invention. En particulier, le corps articulaire, les moyens d'ancrage, les moyens de blocage et/ou les moyens d'ajustement peuvent être conformées différemment des figures.

En outre, les caractéristiques techniques des différents modes de réalisation peuvent être, en totalité ou pour certaines d'entre elles, combinées entre elles. Ainsi, l'implant 1 peut être adapté en termes de coût, de fonctionnalité et de performance.

## Revendications

1. Implant glénoïdien (1) pour prothèse d'épaule, destiné à être implanté dans la glène (B) d'un patient et comprenant :
- un corps articulaire (10) pour l'articulation de l'implant glénoïdien, (1) avec l'humérus prothésé ou non-prothésé ;
- une platine (20; 120; 220 ; 320) de support du corps articulaire (10), cette platine (20 ; 120 ; 220 ; 320) comprenant une surface externe tronconique (21) reliant un côté arrière (22) et un côté avant (23) de la platine et prévue pour coopérer avec une surface interne (14) du corps articulaire (10) ;
- des moyens (50) d'ancrage suivant un axe d'ancrage (X50), adaptés pour être fixés à demeure à la glène (B) ; et
- des moyens (40, 80) de blocage en position de la platine (20 ; 120 ; 220 ; 320) par rapport aux moyens d'ancrage (50) et à la glène (B) ;
**caractérisé en ce que** la platine (20 ; 120 ; 220 ; 320) comprend des moyens (30 ; 230 ; 330) d'ajustement en position de la platine (20 ; 120 ; 220 ; 320) par rapport aux moyens d'ancrage (50), préalablement à son blocage en position par les moyens de blocage (40, 80), sélectivement en rotation autour de l'axe d'ancrage (X50) et en translation transversalement à l'axe d'ancrage (X50).

2. Implant glénoïdien (1) selon la revendication 1, **caractérisé en ce que** les moyens d'ajustement (30 ; 230 ; 330) définissent de plus une liaison rotule pour ajuster en position la platine (20 ; 120 ; 220 ; 320), préalablement à son blocage en position par les moyens de blocage (40, 80), en basculement par rapport à l'axe d'ancrage (X50).

3. Implant glénoïdien (1) selon l'une des revendications 1 ou 2, **caractérisé en ce que** les moyens d'ajustement (30 ; 230 ; 330) comprennent une rainure (30 ; 230; 330) ménagée dans la platine (20 ; 120 ; 220 , 320) suivant une direction (A30) transverse à l'axe d'ancrage (X50) et adaptée pour ajuster en position la platine (20 ; 120 ; 220 ; 320) transversalement à l'axe d'ancrage (X50) indépendamment d'une rotation de cette platine (20 ; 120 ; 220 ; 320) autour de l'axe d'ancrage (X50).

4. Implant glénoïdien (1) selon la revendication 3, **caractérisé en ce que** la rainure (30) comporte au moins deux empreintes (31, 32, 33) délimitant chacune une position distincte de la platine (20 ; 120) transversalement à l'axe d'ancrage (X50) indépendamment d'une rotation de cette platine (20 ; 120) autour de l'axe d'ancrage (X50).

5. Implant glénoïdien (1) selon l'une des revendications précédentes, **caractérisé en ce que** les moyens de blocage (40, 80) comprennent un pion (40) qui coopère avec les moyens d'ancrage (50) et avec les moyens d'ajustement (30 ; 230 ; 330) de la platine (20 ; 120 ; 220 ; 320) pour ajuster en position cette platine (20 ; 120 ; 220 ; 320) par rapport à l'axe d'ancrage (X50).

6. Implant glénoïdien (1) selon les revendications 3 et 5, **caractérisé en ce que** le pion (40) coopère avec la rainure (30 ; 230 ; 330) ménagée dans la platine (20 ; 120 ; 220 ; 320) pour ajuster en position cette platine (20 ; 120 ; 220 ; 320) par rapport à l'axe d'ancrage (X50).

7. Implant glénoïdien (1) selon les revendications 4 et 6, **caractérisé en ce que** le pion (40) reçu dans l'une des empreintes (31, 32, 33) formées dans la rainure (30) bloque en position la platine (20 ; 120) transversalement à l'axe d'ancrage (X50) indépendamment d'une rotation de cette platine (20 ; 120) autour de l'axe d'ancrage (X50).

8. Implant glénoïdien (1) selon l'une des revendications précédentes, **caractérisé en ce que** le corps articulaire (10) présente sélectivement une configuration glénoïdienne anatomique ou inversée.

9. Kit chirurgical adapté pour la mise en oeuvre d'un implant glénoïdien (1) pour prothèse d'épaule selon l'une des revendications 1 à 8, l'implant glénoïdien (1) étant destiné à être implanté dans la glène (B) d'un patient, le kit comprenant :
- au moins un corps articulaire (10) pour l'articulation de l'implant glénoïdien (1) avec l'humérus prothésé ou non-prothésé ;
- au moins une platine (20 ; 120 ; 220, 320) de support du corps articulaire (10), cette platine (20 ; 120 ; 220 ; 320) comprenant une surface externe tronconique (21) reliant un côté arrière (22) et un côté avant (23) de la platine et prévue pour coopérer avec une surface interne (14) du corps articulaire (10) ;
- des moyens (50) d'ancrage suivant un axe d'ancrage (X50), adaptés pour être fixés à demeure à la glène (B) ; et
- des moyens (40, 80) de blocage en position de la platine (20) par rapport aux moyens d'ancrage (50) et à la glène (B) ;
**caractérisé en ce que** la platine (20 ; 120 ; 220 ; 320) comprend des moyens (30 ; 230 ; 330) d'ajustement en position de l'au moins une platine (20 ; 120 ; 220 ; 320) par rapport aux moyens d'ancrage (50), préalablement à son blocage en position par les moyens de blocage (40, 80), sélectivement en rotation autour de l'axe d'ancrage (X50) et transversalement à l'axe d'ancrage (X50).

10. Kit chirurgical selon la revendication 9, **caractérisé en ce que** les moyens d'ajustement (30 ; 230 ; 330) comprennent une rainure (30 ; 230 ; 330) ménagée dans la ou au moins l'une des platines (20 ; 120 ; 220 ; 320) suivant une direction (A30) transverse à l'axe d'ancrage (X50) et adaptée pour ajuster en position cette platine (20 ; 120 ; 220 ; 320) transversalement à l'axe d'ancrage (X50) indépendamment d'une rotation de cette platine (20 ; 120 ; 220 ; 320) autour de l'axe d'ancrage (X50).

## Patentansprüche

1. Gelenkimplantat (1) für eine Schulterprothese, welches bestimmt ist, in der Gelenkpfanne (B) eines Patienten implantiert zu sein und welches aufweist:
- einen Gelenkkörper (10) zur Gelenkverbindung des Gelenkimplantats (1) zusammen mit einer Oberarmknochenprothese oder dem Oberarmknochen ohne Prothese,
- eine Platte (20; 120; 220; 320) zum Stützen des Gelenkkörpers (10), wobei diese Platte (20; 120; 220; 320) eine kegelförmige Außenfläche (21) aufweist, die eine Hinterseite (22) und eine Vorderseite (23) der Platte verbindet und die vorgesehen ist, um mit einer Innenfläche (14) des Gelenkkörpers (10) zu kooperieren,
- Mittel (50) zum Verankern entlang einer Verankerungsachse (X50), welche angepasst sind, um dauerhaft an der Gelenkpfanne (B) fixiert zu sein, und
- Mittel (40, 80) zum Blockieren der Platte (20; 120; 220; 320) in Position bezüglich der Mittel (50) zum Verankern und der Gelenkpfanne (B);
**dadurch gekennzeichnet, dass** die Platte (20; 120; 220; 320) Mittel (30; 230; 330) aufweist zum Einstellen der Position der Platte (20; 120; 220; 320) bezüglich der Mittel zum Verankern (50), vor ihrem Blockieren in Position durch die Mittel zum Blockieren (40, 80), selektiv durch Rotation um die Verankerungsachse (X50) herum und durch Translation quer zur Verankerungsachse (X50).

2. Gelenkimplantat (1) gemäß Anspruch 1, **dadurch gekennzeichnet, dass** die Mittel zum Einstellen (30; 230; 330) ferner eine Kugelgelenkverbindung definieren zum Einstellen der Position der Platte (20; 120; 220; 320), vor ihrem Blockieren in Position durch die Mittel zum Blockieren (40, 80), durch Verschwenkung bezüglich der Verankerungsachse (X50).

3. Gelenkimplantat (1) gemäß einem der Ansprüche 1 oder 2, **dadurch gekennzeichnet, dass** die Mittel (30; 230; 330) zum Einstellen eine Nut (30; 230; 330) aufweisen, welche in der Platte (20; 120; 220; 320) entlang einer Richtung (A30) quer zur Verankerungsachse (X50) vorgesehen ist und angepasst ist, um die Position der Platte (20; 120; 220; 320) quer zur Verankerungsachse (X50) unabhängig von einer Drehung dieser Platte (20; 120; 220; 320) um die Verankerungsachse (X50) herum einzustellen.

4. Gelenkimplantat (1) gemäß Anspruch 3, **dadurch gekennzeichnet, dass** die Nut (30) zumindest zwei Vertiefungen (31, 32, 33) aufweist, welche jede eine individuelle Position der Platte (20; 120) quer zur Verankerungsachse (X50) unabhängig von einer Drehung dieser Platte (20; 120) um die Verankerungsachse (X50) herum begrenzt.

5. Gelenkimplantat (1) gemäß einem der vorhergehenden Ansprüche, **dadurch gekennzeichnet, dass** die Mittel zum Blockieren (40, 80) einen Zapfen (40) aufweisen, welcher mit den Mitteln zum Verankern (50) und mit den Mitteln zum Einstellen (30; 230; 330) der Platte (20; 120; 220; 320) zum Einstellen der Position dieser Platte (20; 120; 220; 320) bezüglich der Verankerungsachse (X50) kooperiert.

6. Gelenkimplantat (1) gemäß den Ansprüchen 3 und 5, **dadurch gekennzeichnet, dass** der Zapfen (40) mit der Nut (30; 230; 330) kooperiert, die in der Platte (20; 120; 220; 320) zum Einstellen der Position dieser Platte (20; 120; 220; 320), bezüglich der Verankerungsachse (X50) vorgesehen ist.

7. Gelenkimplantat (1) gemäß den Ansprüchen 4 und 6, **dadurch gekennzeichnet, dass** der Zapfen (40), welcher in einer der Vertiefungen (31, 32, 33) aufgenommen ist, die in der Nut (30) geformt sind, die Platte (20; 120) in Position quer zur Verankerungsachse (X50) unabhängig von einer Drehung dieser Platte (20; 120) um die Verankerungsachse (X50) herum blockiert.

8. Gelenkimplantat (1) gemäß einem der vorhergehenden Ansprüche, **dadurch gekennzeichnet, dass** der Gelenkkörper (10) selektiv eine anatomische Schultergelenkkonfiguration oder eine invers-anatomische Schultergelenkkonfiguration aufweist.

9. Chirurgisches Kit, welches zum Umsetzen eines Gelenkimplantats (1) für eine Schulterprothese gemäß einem der Ansprüche 1 bis 8 eingerichtet ist, wobei das Gelenkimplantat (1) bestimmt ist, in der Gelenkpfanne (B) eines Patienten implantiert zu sein, wobei das Kit aufweist:
- zumindest einen Gelenkkörper (10) zur Gelenkverbindung des Gelenkimplantats (1) mit einer Oberarmknochenprothese oder dem Oberarmknochen ohne Prothese,
- zumindest eine Platte (20; 120; 220; 320) zum Stützen des Gelenkkörpers (10), wobei diese Platte (20; 120; 220; 320) eine konische Außenfläche (21) aufweist, die eine Hinterseite (22) und eine Vorderseite (23) der Platte verbindet und die vorgesehen ist, um mit einer Innenfläche (14) des Gelenkkörpers (10) zu kooperieren,
- Mittel (50) zum Verankern entlang einer Verankerungsachse (X50), welche angepasst sind, um dauerhaft an der Gelenkpfanne (B) fixiert zu sein, und
- Mittel (40, 80) zum Blockieren der Platte (20) in Position bezüglich der Mittel zum Verankern (50) und der Gelenkpfanne (B) ;
**dadurch gekennzeichnet, dass** die Platte (20; 120; 220; 320) Mittel (30; 230; 330) aufweist zum Einstellen der zumindest einen Platte (20; 120; 220; 320) in Position bezüglich der Mittel zum Verankern (50), vor ihrem Blockieren in Position durch die Mittel zum Blockieren (40, 80), selektiv durch Drehung um die Verankerungsachse (X50) und quer zur Verankerungsachse (X50).

10. Chirurgisches Kit gemäß Anspruch 9, **dadurch gekennzeichnet, dass** die Mittel zum Einstellen (30; 230; 330) eine Nut (30; 230; 330) aufweisen, welche in der oder in zumindest einer der Platten (20; 120; 220; 320) entlang einer Richtung (A30) quer zur Verankerungsachse (X50) vorgesehen ist und angepasst ist, um die Position dieser Platte (20; 120; 220; 320) quer zur Verankerungsachse (X50) unabhängig von einer Drehung dieser Platte (20; 120; 220; 320) um die Verankerungsachse (X50) herum einzustellen.

## Claims

1. A glenoidal implant (1) for a shoulder prosthesis, designed to be implanted in the glenoid (B) of a patient and comprising:
- an articular body (10) for articulating the glenoidal implant (1) with the humerus provided with or without a prosthesis;
- a plate (20; 120; 220; 320) for supporting the articular body (10), this plate (20; 120; 220; 320) comprising an outer frustoconical surface (21) that connects a rear side (22) and a front side (23) of the plate and that is provided for cooperating with an inner surface (14) of the articular body (10);
- means (50) for anchoring along an anchor axis (X50), suitable for being permanently fastened to the glenoid (B); and
- means (40, 80) for locking the plate (20; 120; 220; 320) in position relative to the anchoring means (50) and the glenoid (B);
**characterized in that** the plate comprises means (30; 230; 330) for adjusting the position of the plate (20; 120; 220; 320) relative to the anchoring means, before it is locked in position by the locking means (40, 80), selectively in rotation around the anchoring axis (X50) and in translation transverse to the anchoring axis (X50).

2. The glenoidal implant (1) according to claim 1, **characterized in that** the adjustment means (30; 230; 330) define a ball-type connection for adjusting the position of the plate (20; 120; 220; 320), before it is locked in position by the locking means (40, 80), in terms of tilt relative to the anchoring axis (X50).

3. The glenoidal implant (1) according to one of claims 1 or 2, **characterized in that** the adjustment means (30; 230; 330) comprise a groove (30; 230; 330) formed in the plate (20; 120; 220; 320) in a direction (A30) transverse to the anchoring axis (X50) and suitable for adjusting the position of the plate (20; 120; 220; 320) transverse to the anchoring axis (X50) independently of a rotation of that plate (20; 120; 220; 320) around the anchoring axis (X50).

4. The glenoidal implant (1) according to claim 3, **characterized in that** the groove (30) comprises at least two cavities (31, 32, 33) each delimiting a distinct position of the plate (20; 120) transverse to the anchoring axis (X50) independently of a rotation of that plate (20; 120) around the anchoring axis (X50).

5. The glenoidal implant (1) according to one of the preceding claims, **characterized in that** the locking means (40, 80) comprise a pin (40) that cooperates with the anchoring means (50) and with the adjustment means (30; 230; 330) of the plate (20; 120; 220; 320), to adjust the position of that plate (20; 120; 220; 320) relative to the anchoring axis (X50).

6. The glenoidal implant (1) according to claims 3 and 5, **characterized in that** the pin (40) cooperates with the groove (30; 230; 330) formed in the plate (20; 120; 220; 320), to adjust the position of that plate (20; 120; 220; 320) relative to the anchoring axis (X50).

7. The glenoidal implant (1) according to claims 4 and 6, **characterized in that** the pin (40) received in one of the cavities (31, 32, 33) formed in the groove (30) locks the position of the plate (20 ; 120) transversally to the anchoring axis (X50) independently of a rotation of that plate (20; 120) around the anchoring axis (X50).

8. The glenoidal implant (1) according to one of the preceding claims, **characterized in that** the articular body (10) selectively has an anatomical or reversed glenoidal configuration.

9. A surgical kit suitable for implementing a glenoidal implant (1) for a shoulder prosthesis according to one of claims 1 to 8, the glenoidal implant (1) being designed to be implanted in the glenoid (B) of a patient, the kit comprising:
- at least one articular body (10) for articulating the glenoidal implant (1) with the humerus provided with or without a prosthesis;
- at least one plate (20; 120; 220; 320) for supporting the articular body (10), this plate (20; 120; 220; 320) comprising an outer frustoconical surface (21) that connects a rear side (22) and a front side (23) of the plate and that is provided for cooperating with an inner surface (14) of the articular body (10);
- means (50) for anchoring along an anchor axis (X50), suitable for being permanently fastened to the glenoid (B); and
- means (40, 80) for locking the plate (20) in position relative to the anchoring means (50) and the glenoid (B);
**characterized in that** the plate comprises means (30; 230; 330) for adjusting the position of the at least one plate (20; 120; 220; 320) relative to the anchoring means, before it is locked in position by the locking means (40, 80), selectively in rotation around the anchoring axis (X50) and in translation transverse to the anchoring axis (X50).

10. The surgical kit according to claim 9, **characterized in that** the adjustment means (30; 230; 330) comprise a groove (30; 230; 330) formed in the or at least one of the plates (20; 120; 220; 320) in a direction (A30) transverse to the anchoring axis (X50) and suitable for adjusting the position of the plate (20; 120; 220; 320) transverse to the anchoring axis (X50) independently of a rotation of that plate (20; 120; 220; 320) around the anchoring axis (X50).
